# FASCICULE DE BREVET EUROPEEN

(11) **EP 3 435 285 B1**
(45) Date de publication et mention de la délivrance du brevet: **11.03.2020**
(21) Numéro de dépôt: 18184886.2
(22) Date de dépôt: 23.07.2018
(51) Int. Cl.: G06K 9/00

(54) **CAPTEUR THERMIQUE AVEC DEUX PORTIONS PYROÉLECTRIQUES SUPERPOSÉES, POUR LA MESURE D'UN DIFFÉRENTIEL DE CHARGES**
THERMISCHER SENSOR MIT ZWEI ÜBEREINANDER ANGEORDNETEN PYROELEKTRISCHEN ABSCHNITTEN FÜR DIE MESSUNG EINES LADUNGSUNTERSCHIEDS
THERMAL SENSOR WITH TWO SUPERIMPOSED PYROELECTRIC PORTIONS, FOR MEASURING A LOAD DIFFERENTIAL

(30) Priorité: 24.07.2017 FR 1757000
(43) Date de publication de la demande: 30.01.2019
(73) Titulaire: Commissariat à l'énergie atomique et aux énergies alternatives, 75015 Paris (FR); Idemia Identity & Security France, 92400 Courbevoie (FR)
(72) Inventeur: MAINGUET, Jean-François, 38100 GRENOBLE (FR); FOURRE, Joël Yann, 78160 MARLY LE ROI (FR)
(74) Mandataire: Brevalex

(56) Documents cités:
- WO-A1-2017/093252
- FR-A1- 3 044 407
- FR-A1- 3 044 408
- US-A- 6 061 464

## Description

### DOMAINE TECHNIQUE

L'invention se rapporte à un capteur de motifs thermiques, exploitant les propriétés de pyroélectricité d'un matériau, et formant avantageusement un capteur d'empreinte papillaire, notamment d'empreinte digitale.

### ÉTAT DE LA TECHNIQUE ANTÉRIEURE

Dans l'art antérieur, un tel capteur de motifs thermiques comporte une matrice de capacités pyroélectriques.

Chaque capacité pyroélectrique comporte une portion en matériau pyroélectrique, disposée entre une électrode inférieure et une électrode supérieure. Une électrode est portée à un potentiel constant, et forme une électrode de référence. L'autre électrode collecte des charges pyroélectriques, générées par le matériau pyroélectrique en réponse à une variation de température.

Chaque capacité pyroélectrique forme ainsi un transducteur, pour traduire une variation temporelle de température en un signal électrique tel qu'une différence de potentiels électriques.

Un tel capteur est par exemple de type passif, exploitant uniquement une différence entre une température sur le motif thermique à imager, et une température du capteur.

Dans le cas particulier d'un capteur d'empreinte papillaire, comportant une surface de contact pour y appliquer l'empreinte à imager, un tel capteur exploite plus particulièrement une différence de température entre le doigt et la surface de contact du capteur. Un tel capteur est décrit par exemple dans le brevet US 4,394,773.

Au niveau des crêtes de l'empreinte, le doigt est en contact physique direct avec le capteur. Un transfert thermique entre la peau et la surface de contact du capteur s'effectue par conduction, ce qui conduit à une première variation temporelle de température.

Au niveau des vallées de l'empreinte, le doigt n'est pas en contact physique direct avec le capteur. Un transfert thermique entre la peau et la surface de contact du capteur s'effectue à travers l'air qui est plutôt un isolant thermique, ce qui conduit à une seconde variation temporelle de température, moins importante.

La différence entre ces deux variations temporelles de température se traduit par une différence entre des signaux mesurés par les capacités pyroélectriques, selon qu'elles se trouvent sous une vallée ou sous une crête de l'empreinte. L'image de l'empreinte présente donc un contraste qui dépend de cette différence.

Après quelques secondes à peine, la température du doigt et la température de la surface de contact du capteur s'homogénéisent, et il n'est plus possible d'obtenir un contraste satisfaisant.

Un autre type de capteur, de type actif, offre une solution à ce problème grâce à l'ajout de moyens de chauffage sous la surface de contact du capteur. Un tel capteur est décrit par exemple dans la demande de brevet EP 2 385 486 A1. Un autre exemple d'art antérieur est WO 2017/093252.

Les moyens de chauffage dissipent une certaine quantité de chaleur dans chaque pixel du capteur. La variation de température se rapporte donc à la mesure dans laquelle cette quantité de chaleur est évacuée du pixel. La variation de température est donc importante au niveau des vallées de l'empreinte, où la chaleur n'est transférée au doigt qu'à travers l'air, et plus faible au niveau des crêtes de l'empreinte, où la chaleur est transférée efficacement au doigt, par conduction.

Les moyens de chauffage permettent donc de briser un équilibre thermique, pouvant s'établir entre la surface de contact du capteur et le doigt. Cela permet d'améliorer et de conserver au cours du temps, le contraste d'une image acquise à l'aide dudit capteur.

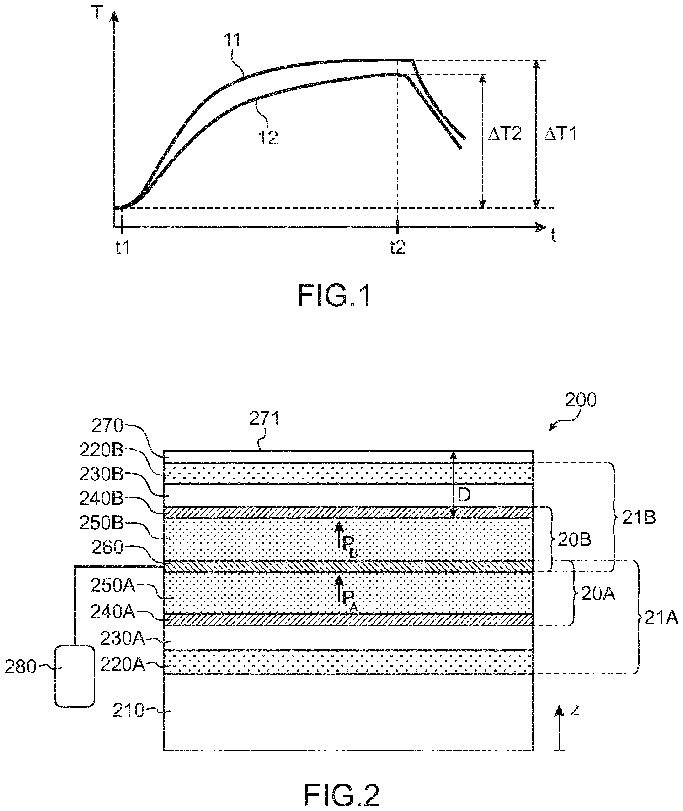

La figure 1 illustre de façon schématique des courbes de température en fonction du temps, au niveau d'une vallée (courbe 11), respectivement au niveau d'une crête (courbe 12).

La température est mesurée à un instant t1, au démarrage du chauffage, et à un instant t2, après une certaine durée d'activation du chauffage.

Au niveau d'une vallée, on mesure une variation de température ΔT1.

Au niveau d'une crête, on mesure une variation de température ΔT2.

Le contraste de l'image ainsi obtenue est assez faible.

Un objectif de la présente invention est de proposer un capteur pyroélectrique, en particulier un capteur de type thermique actif, offrant un meilleur contraste que dans l'art antérieur, notamment un contraste supérieur à 20%.

### EXPOSÉ DE L'INVENTION

Cet objectif est atteint avec un capteur de motifs thermiques, de type capteur pyroélectrique, comportant une surface de contact pour y appliquer un objet à imager, en particulier une empreinte papillaire, et une pluralité de pixels répartis entre un substrat et ladite surface de contact.

Selon l'invention, chaque pixel comprend :
- une première structure, comportant une première portion comprenant un matériau pyroélectrique, ladite première structure étant en contact physique direct avec une électrode de collecte de charges ;
- une deuxième structure, comportant une deuxième portion comprenant un matériau pyroélectrique, ladite deuxième structure étant en contact physique direct avec une électrode de collecte de charges, la première structure, la deuxième structure, et l'au moins une électrode de collecte de charge étant superposées au-dessus du substrat ;
- au moins un élément de chauffage, pour chauffer la première et la deuxième portions comprenant un matériau pyroélectrique ; et
- un dispositif électronique connecté à l'au moins une électrode de collecte de charges, et configuré pour mesurer une différence entre des charges générées par l'une parmi la première et la deuxième portions comprenant un matériau pyroélectrique, et des charges générées par l'autre parmi la première et la deuxième portions comprenant un matériau pyroélectrique.

La première et la deuxième structures sont superposées, et chacune de ces deux structures comporte une portion distincte comprenant un matériau pyroélectrique.

Ainsi, l'une des portions comprenant un matériau pyroélectrique s'étend du côté du substrat, tandis que l'autre des portions comprenant un matériau pyroélectrique s'étend du côté de l'objet à imager, le cas échéant du côté d'une surface de contact.

La portion comprenant un matériau pyroélectrique qui s'étend du côté du substrat échange peu voire pas du tout de chaleur avec l'objet. Elle forme une portion dite de calibration.

Lorsque l'au moins un élément de chauffage chauffe cette portion, la chaleur apportée par l'élément de chauffage n'est pas transférée à l'objet.

La variation de température obtenue, dans ladite portion comprenant un matériau pyroélectrique, se traduit par la génération de charges pyroélectriques dites de calibration, dont la quantité dépend simplement du chauffage apporté par l'au moins un élément de chauffage.

Ces charges de calibration sont collectées par l'au moins une électrode de collecte de charges.

En revanche, la portion comprenant un matériau pyroélectrique qui s'étend du côté de l'objet à imager échange de la chaleur avec cet objet. Elle forme une portion dite de mesure.

L'au moins un élément de chauffage est agencé au-dessus du substrat. Il est donc superposé, au-dessus de ce substrat, avec la première structure, la deuxième structure, et l'au moins une électrode de collecte de charge.

Lorsque l'au moins un élément de chauffage chauffe cette portion, la chaleur apportée par l'élément de chauffage est transférée plus ou moins efficacement à l'objet en fonction des caractéristiques de ce dernier. Dans le cas d'un capteur d'empreinte, elle est transférée plus ou moins efficacement au doigt selon que le pixel est surmonté par une crête ou par une vallée de l'empreinte.

La variation de température obtenue, dans ladite portion comprenant un matériau pyroélectrique, se traduit par la génération de charges pyroélectriques, dite de mesure. Leur quantité dépend à la fois du chauffage apporté par l'au moins un élément de chauffage, et des caractéristiques de l'objet à imager.

Ces charges de mesure sont collectées par l'au moins une électrode de collecte de charges.

En mesurant une différence entre les charges de mesure et les charges de calibration (ou inversement), on peut, dans un cas idéal, obtenir une quantité de charges pyroélectriques liée uniquement aux caractéristiques de l'objet à imager.

Dans tous les cas, on peut s'affranchir au moins partiellement d'une partie non utile de signal, ce qui permet d'améliorer le contraste de l'image de l'objet.

Ladite différence de charges est mesurée, et non calculée. En d'autres termes, on mesure directement une quantité de charges moindre, ce qui permet d'utiliser en détection des amplificateurs à gain important, sans risque de saturer le signal.

Le capteur obtenu est donc plus sensible, ce qui permet de chauffer moins les pixels pour la détection active et donc de consommer moins d'énergie, et/ou d'utiliser des couches de protection plus épaisses pour protéger le capteur, et/ou de diminuer une durée des mesures de variations de température.

La solution proposée présente l'avantage de ne pas impliquer la mise en œuvre de calculs lourds, susceptibles de complexifier la réalisation du capteur selon l'invention.

En outre, tous les pixels du capteur peuvent être strictement identiques, ce qui simplifie encore sa fabrication.

La solution proposée offre également une très grande précision, les données de calibration étant, par construction, obtenues simultanément aux données de mesure. On évite ainsi d'éventuelles imprécisions de calibration liées à des dérives temporelles du capteur (par exemple usure d'une surface de contact). On s'affranchit également de l'effet de variations du chauffage, d'une mesure à l'autre, de sorte que l'invention ne requiert pas un contrôle extrêmement précis du chauffage.

La solution proposée offre également une très grande précision en ce que chaque pixel possède ses propres données de calibration. On peut ainsi s'affranchir d'éventuelles différences de sensibilité entre les pixels, liées par exemple à des différences d'usure et/ou de chauffage.

Le capteur selon l'invention peut être dit « équilibré », en ce qu'il y a un équilibrage des charges pour les rapprocher de la valeur nulle, idéalement atteinte lorsqu'aucun objet ne touche le capteur.

Cet équilibrage des charges permet de mieux exploiter une dynamique de numérisation d'un signal fourni par le dispositif électronique.

De préférence, la première portion comprenant un matériau pyroélectrique et la deuxième portion comprenant un matériau pyroélectrique présentent une même épaisseur et une même composition chimique.

Selon un premier mode de réalisation, la première et la deuxième structures sont chacune en contact physique direct avec la même électrode de collecte de charges, dite électrode commune, située entre la première et la deuxième structures.

Selon ce premier mode de réalisation, la première et la deuxième portions comprenant un matériau pyroélectrique peuvent présenter chacune une polarisation respective, ces polarisations étant orientées dans la même direction et le même sens.

Selon ce premier mode de réalisation, ledit dispositif électronique est avantageusement configuré pour mesurer des charges collectées par ladite électrode commune.

Chaque pixel peut comporter également deux électrodes de référence, situées de part et d'autre d'un empilement comportant la première structure, l'électrode commune, et la deuxième structure.

Selon un deuxième mode de réalisation, la première structure et la deuxième structure sont chacune en contact physique direct avec deux électrodes respectives de collecte de charges, l'une étant située d'un côté d'un empilement comprenant la première et la deuxième structures, et l'autre étant située de l'autre côté de cet empilement.

Selon ce deuxième mode de réalisation, la première et la deuxième portions comprenant un matériau pyroélectrique peuvent présenter chacune une polarisation respective, ces polarisations étant orientées dans des sens opposés.

Selon ce deuxième mode de réalisation, ledit dispositif électronique comprend avantageusement un amplificateur différentiel, connecté en entrée à l'une et à l'autre des deux électrodes de collecte de charges.

Chaque pixel peut comporter également une électrode de référence, située entre la première et la deuxième structures.

L'au moins un élément de chauffage peut comprendre un élément de chauffage en matériau électriquement conducteur, situé entre la première et la deuxième structures.

En variante, dans chacun de ces deux modes de réalisation, l'au moins un élément de chauffage peut comprendre deux éléments de chauffage en matériau électriquement conducteur, situés de part et d'autre d'un empilement comportant la première et la deuxième structures.

Les éléments de chauffage des différents pixels peuvent former ensemble des bandes chauffantes, réparties de part et d'autre dudit empilement, et disposées deux à deux face à face.

Chaque pixel peut comporter également une portion de colle, située entre les première et deuxième structures.

### BRÈVE DESCRIPTION DES DESSINS

La présente invention sera mieux comprise à la lecture de la description d'exemples de réalisation donnés à titre purement indicatif et nullement limitatif, en faisant référence aux dessins annexés sur lesquels :
- la figure 1 illustre de manière schématique des courbes de température dans un capteur d'empreinte de type thermique actif ;
- la figure 2 illustre un premier mode de réalisation d'un capteur de motifs thermiques selon l'invention ;
- la figure 3 illustre de manière schématique des courbes de température dans un capteur de motifs thermiques selon l'invention ;
- la figure 4 illustre une variante du premier mode de réalisation d'un capteur de motifs thermiques selon l'invention ;
- les figures 5 à 7 illustrent trois variantes d'un deuxième mode de réalisation d'un capteur de motifs thermiques selon l'invention ; et
- la figure 8 illustre de manière schématique, selon une vue de dessus, un agencement avantageux des électrodes de collecte de charges et des éléments de chauffage, dans un capteur de motifs thermiques selon l'invention.

### EXPOSÉ DÉTAILLÉ DE MODES DE RÉALISATION PARTICULIERS

Dans la suite, on se place plus particulièrement, et de manière non limitative, dans le cadre d'une détection d'empreinte papillaire, notamment une empreinte digitale.

L'invention n'est pas limitée à cet exemple d'application. Le capteur de motifs thermiques selon l'invention peut permettre d'imager d'autres types d'objets, par exemple des circuits imprimés.

Le capteur de motifs thermiques selon l'invention peut également former un appareil d'analyse de type spectromètre de masse, ou débitmètre, permettant de réaliser des mesures en diverses profondeurs dans un objet, et d'étudier la réponse de l'objet à un chauffage connu.

Dans la description qui suit, les termes « sur », « au-dessus », « supérieur », « sous », « dessous », « inférieur », se rapportent à l'orientation des figures correspondantes.

Pour plus de clarté, on a représenté sur certaines des figures les axes (Ox), (Oy) et/ou (Oz) d'un repère orthonormé.

La figure 2 illustre un premier mode de réalisation d'un capteur selon l'invention.

On a représenté plus particulièrement un pixel 200 de ce capteur, les différents pixels étant ici formés, et répartis selon une matrice en deux dimensions au-dessus d'un substrat commun 210.

Le substrat 210 est par exemple un substrat ultra-mince en plastique ou en verre, d'épaisseur par exemple inférieure à 12 µm. Il peut également s'agir d'un substrat en poly(téréphtalate d'éthylène) (PET), d'épaisseur comprise entre 100 µm et 150 µm, par exemple 125 µm.

Chaque pixel du capteur comprend, superposés dans cet ordre au-dessus du substrat 210 :
- une première résistance électrique 220A, adaptée à être connectée à une source de courant ou de tension, et formant un élément de chauffage disposé du côté du substrat 210 ;
- une première couche en matériau diélectrique 230A, présentant à la fois des propriétés d'isolant électrique et de conducteur thermique ;
- une première électrode de référence 240A, connectée à une source de potentiel constant, de préférence à la masse ;
- une première portion 250A comprenant un matériau pyroélectrique ;
- une électrode 260 dite de collecte de charges ;
- une deuxième portion 250B comprenant matériau pyroélectrique ;
- une deuxième électrode de référence 240B, connectée à une source de potentiel constant, de préférence à la masse ;
- une deuxième couche en matériau diélectrique 230B, présentant à la fois des propriétés d'isolant électrique et de conducteur thermique ;
- une deuxième résistance électrique 220B, adaptée à être connectée à une source de courant ou de tension, et formant un élément de chauffage disposé du côté opposé au substrat 210 ; et
- une couche de protection 270, optionnelle, par exemple en DLC *(pour « Diamond-Like Carbon »),* pour protéger les couches inférieures à l'égard des contacts répétés avec le doigt.

La couche la plus externe du capteur, du côté opposé au substrat, forme une surface de contact 271, pour y appliquer l'objet à imager, ici l'empreinte digitale d'un doigt. Ladite surface de contact 271 s'étend parallèle au plan (Oxy).

La distance D, selon l'axe (Oz), entre la surface de contact 271 et la face supérieure de la deuxième portion 250B comprenant matériau pyroélectrique, est avantageusement inférieure à un pas de répartition des pixels du capteur (le cas échéant inférieure au plus petit pas de répartition des pixels du capteur, si le pas selon (Ox) et le pas selon (Oy) sont différents l'un de l'autre).

Les première et deuxième électrodes de référence peuvent être connectées à la même source de potentiel constant.

Dans tout le texte, lorsqu'on décrit une électrode de référence, on nomme « source de potentiel constant » une source fournissant un potentiel qui reste constant au moins entre un instant de remise à zéro d'un circuit de lecture et un instant de lecture par ledit circuit (circuit de lecture pour mesurer une quantité de charges, et comportant au moins le dispositif électronique tel que décrit dans la suite).

Ici, la deuxième électrode de référence 240B forme également un blindage électromagnétique pour protéger le capteur à l'égard des parasites électrostatiques, notamment autour de 50Hz, apportés par le contact avec la peau lorsque le doigt touche la surface de contact du capteur. Afin d'optimiser ce blindage, la deuxième électrode de référence 240B s'étend d'un seul tenant et sans ouverture au-dessus du substrat, en passant par l'ensemble des pixels du capteur.

La première portion 250A comprenant un matériau pyroélectrique s'étend ici en contact physique direct, côté inférieur avec la première électrode de référence 240A, côté supérieur avec l'électrode 260 de collecte de charges.

Le cas échéant, une ou plusieurs couches intercalaires conductrices d'électricité peuvent s'insérer entre ladite première portion 250A et l'électrode 260 de collecte de charges, et/ou entre ladite première portion 250A et la première électrode de référence 240A. On définit ici une première structure, comportant au moins la première portion 250A comprenant un matériau pyroélectrique, et, le cas échéant, cette ou ces couche(s) intercalaire(s).

La première structure, la première électrode de référence 240A et l'électrode 260 de collecte de charges, forment ensemble une première capacité pyroélectrique 20A, située du côté du substrat.

De la même façon, la deuxième portion 250B comprenant un matériau pyroélectrique s'étend ici en contact physique direct, côté inférieur avec l'électrode 260 de collecte de charges, et côté supérieur avec la deuxième électrode de référence 240B.

Le cas échéant, une ou plusieurs couches intercalaires conductrices d'électricité peuvent s'insérer entre ladite deuxième portion 250B et l'électrode 260 de collecte de charges, et/ou entre ladite deuxième portion 250B et la deuxième électrode de référence 240B. On définit ici une deuxième structure, comportant au moins la deuxième portion 250B comprenant un matériau pyroélectrique, et, le cas échéant, cette ou ces couche(s) intercalaire(s).

La deuxième structure, la deuxième électrode de référence 240B et l'électrode 260 de collecte de charges, forment ensemble une deuxième capacité pyroélectrique 20B, située du côté de la surface de contact 271.

L'électrode 260 de collecte de charges est donc commune aux deux capacités pyroélectriques 20A, 20B.

La première capacité pyroélectrique 20A est chauffée par la première résistance électrique 220A, par l'intermédiaire de la première couche en matériau diélectrique 230A, pour la mise en œuvre d'une détection de type actif.

La première capacité pyroélectrique 20A, la première résistance électrique 220A, et la première couche en matériau diélectrique 230A forment ensemble une première cellule de détection thermique 21A, située du côté du substrat.

De façon similaire, la deuxième capacité pyroélectrique 20B est chauffée par la deuxième résistance électrique 220B, par l'intermédiaire de la deuxième couche en matériau diélectrique 230B, pour la mise en œuvre d'une détection de type actif.

La deuxième capacité pyroélectrique 20B, la deuxième résistance électrique 220B, et la deuxième couche en matériau diélectrique 230B forment ensemble une deuxième cellule de détection thermique 21B, située du côté de la surface de contact, et superposée à la première cellule 21A.

L'agencement de ces deux cellules 21A, 21B présente une symétrie, relativement à un plan parallèle à la surface de contact du capteur, passant ici par l'électrode 260 de collecte de charges.

En particulier, les dimensions et compositions des différents éléments constitutifs de chaque cellule 21A, 21B sont ici deux à deux identiques. Plus particulièrement, les deux portions 250A, 250B comprenant un matériau pyroélectrique présentent la même épaisseur (dimension selon l'axe z) et la même composition. Elles peuvent être en particulier constituées d'un même matériau ou mélange de matériaux.

Ainsi, la résistance thermique entre la première résistance électrique 220A et la première portion 250A est égale à la résistance thermique entre la deuxième résistance électrique 220B et la deuxième portion 250B.

En outre, la première résistance électrique 220A et la deuxième résistance électrique 220B sont parcourues ici par un même courant, de sorte qu'elles fournissent chacune une même quantité de chaleur à la première, respectivement la deuxième portion comprenant un matériau pyroélectrique.

La première cellule de détection thermique 21A, située du côté du substrat, est peu voire pas du tout affectée par des échanges thermiques avec un objet appuyé contre la surface de contact 271 du capteur. Ainsi, l'activation de la première résistance électrique 220A entraîne une variation de température ΔT_{A} de la couche 250A, qui ne dépend pas (ou très peu) des caractéristiques de l'objet appuyé contre la surface de contact 271. Elle dépend des caractéristiques thermiques du substrat et de ses éventuelles couches inférieures, supposé homogène sur l'ensemble du capteur. La variation de température ΔT_{A} est donc la même, peu importe que le pixel 200 soit surmonté par une crête ou une vallée d'une empreinte digitale, au niveau de la surface de contact 271. La première cellule 21A forme ainsi une cellule de calibration.

Au contraire, la deuxième cellule de détection thermique 21B, située du côté de la surface de contact 271, est fortement affectée par des échanges thermiques avec un objet appuyé contre la surface de contact 271 du capteur. L'activation de la deuxième résistance électrique 220B entraîne une variation de température ΔT_{B}, qui dépend fortement des caractéristiques de l'objet appuyé contre la surface de contact 271. La variation de température ΔT_{B} n'est donc pas la même, selon que le pixel du capteur est surmonté par une crête ou une vallée d'une empreinte digitale, au niveau de la surface de contact 271. La deuxième cellule 21B forme ainsi une cellule de mesure.

Au niveau de la cellule de calibration 21A, la variation de température ΔT_{A} entraîne la génération de premières charges pyroélectriques, dites de calibration, dont la quantité dépend directement de ΔT_{A} (le nombre de charges générées est proportionnel à la variation de température).

Au niveau de la cellule de mesure 21B, la variation de température ΔT_{B} entraîne la génération de deuxièmes charges pyroélectriques, dites de mesure, dont la quantité dépend directement de ΔT_{B}.

Par conséquent, seule la quantité de charges de mesure varie, selon que le pixel est surmonté par une crête ou une vallée d'une empreinte.

Seule la variation de cette quantité de charges est utile pour imager l'empreinte.

On distingue donc, parmi les charges de mesure, des charges dites utiles, dont la quantité varie selon que le pixel est surmonté par une crête ou une vallée d'une empreinte, les autres charges étant dites inutiles.

L'idée à la base de l'invention consiste à soustraire les charges de calibration aux charges de mesure (ou inversement), afin de s'affranchir le plus possible de ces charges dites inutiles.

Si l'on se reporte de nouveau à la figure 1, il s'agit de s'affranchir, au moins en partie, des portions de signal situées sous la courbe 12. En d'autres termes, plutôt que de mesurer, via des mesures de quantité de charges, une forte variation de température ΔT1 ou ΔT2, on mesure de faibles variations de température, proches de l'écart entre ΔT1 et ΔT2.

On peut par exemple s'affranchir de l'intégralité des portions de signal situées sous la courbe 12 en combinant les charges ΔT_{A} et ΔT_{B}. On mesure alors, via des mesures de quantité de charges, une variation nulle, ou égale à l'écart entre ΔT1 et ΔT2, selon que le pixel est surmonté par une crête ou par une vallée.

On peut même soustraire un peu plus que les charges inutiles, de façon à mesurer une variation de charges positive ou négative, selon que le pixel est surmonté par une crête ou par une vallée.

En d'autres termes, différents types de calibration peuvent être mis en œuvre, par exemple pour supprimer une partie seulement, ou la totalité d'une partie inutile de signal, ou en d'autres termes, pour soustraire la quantité souhaitée de charges de calibration.

On peut ajuster la quantité de charges soustraite par un choix adapté, du côté de la cellule 21A, des caractéristiques thermiques du substrat et d'éventuelle(s) couche(s) inférieure(s) et/ou de la quantité de chaleur injectée par la résistance 220A, des caractéristiques pyroélectriques de la première portion 250A, et/ou de l'épaisseur de la première portion 250A, relativement aux caractéristiques équivalentes du côté de la cellule 21B.

On détaille dans la suite les différents paramètres pouvant être ajustés, pour soustraire la quantité souhaitée de charges de calibration.

Dans tous les cas, on diminue fortement l'amplitude de la plus grande variation de signal mesurée, relativement à une différence entre un signal correspondant aux crêtes et un signal correspondant aux vallées, de sorte que le contraste est augmenté.

Dans le premier mode de réalisation illustré ici, la soustraction de charges est réalisée directement au niveau de l'électrode 260 de collecte de charges, commune ici à la cellule de calibration 21A et à la cellule de mesure 21B.

Les deux portions 250A, 250B comprenant un matériau pyroélectrique sont polarisées, respectivement selon un axe P_{A} et P_{B}.

Les axes P_{A} et P_{B} sont orientés selon une même direction, ici parallèle à l'axe z, où l'axe z est un axe orthogonal à la surface de contact 271 du capteur.

En outre, les axes P_{A} et P_{B} sont orientés ici dans le même sens (de bas en haut, ou de haut en bas).

Ainsi, et du fait de la symétrie entre la cellule de calibration 21A et la cellule de mesure 21B, et de la position centrale de l'électrode 260 de collecte de charges, les charges de mesure ΔT_{B} et les charges de calibration ΔT_{A}, toutes collectées au niveau de l'électrode 260 de collecte de charges, présentent des signes opposés. On réalise ainsi une soustraction de charges en additionnant des charges positives et des charges négatives.

L'électrode 260 de collecte de charges est connectée à un dispositif électronique 280, configuré pour mesurer une quantité de charges. Le dispositif électronique 280 mesure donc, directement, une quantité de charges correspondant à la différence entre les charges générées dans la cellule de mesure 21B, par la deuxième portion 250B comprenant un matériau pyroélectrique, et les charges générées dans la cellule de calibration 21A, par la première portion 250A comprenant un matériau pyroélectrique (ou inversement).

Le dispositif électronique 280 est un dispositif connu, tel que ceux des capteurs pyroélectriques de l'art antérieur. Le dispositif électronique 280 peut notamment mesurer des quantités de charge par lecture de tensions ou de courant.

Afin d'optimiser les performances de la calibration, il est préférable de maximiser une isolation thermique entre les deux portions 250A, 250B comprenant un matériau pyroélectrique.

Ce matériau pyroélectrique est donc avantageusement à base de PVDF (poly(fluorure de vinylidène)), qui est un bon isolant thermique. D'autres matériaux pyroélectriques ne sont cependant pas à exclure, par exemple l'AIN (nitrure d'aluminium). Les première et deuxième portions 250A, 250B peuvent être entièrement constituées de matériau pyroélectrique, ou constituées d'un mélange de plusieurs matériaux dont l'un au moins est pyroélectrique.

Un élément conducteur d'électricité situé entre ces deux portions en matériau pyroélectrique, ici l'électrode 260 de collecte de charges, peut en outre présenter des propriétés d'isolant thermique. Cet élément conducteur est par exemple en PEDOT:PSS (mélange de deux polymères : poly(3,4-ethylenedioxythiophene) et polystyrenesulfonate).

Selon une variante, non représentée, la couche 260 communes aux cellules 21A et 21B, est dupliquée en deux couches 260A, 260B entre lesquelles s'étend une couche d'isolation thermique. Les deux couches 260A, 260B sont reliées électriquement ensemble, par exemple par l'intermédiaire de vias traversant ladite couche d'isolation thermique. Les vias peuvent être répartis sur toute la surface du capteur, ou uniquement en bordure de la matrice de pixels.

Selon une autre variante, le substrat 210 est en silicium. Une couche en matériau électriquement isolant est alors intercalée entre chaque première résistance électrique 220A et le substrat 210. Cette couche intercalée présente avantageusement en outre une résistance thermique élevée, pour limiter l'influence du silicium qui est un excellent conducteur thermique.

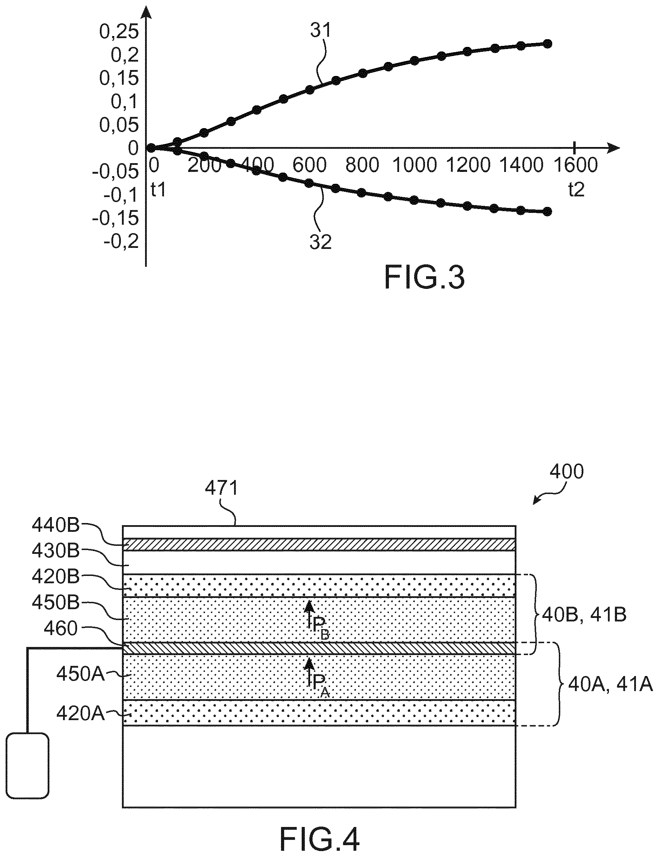

La figure 3 illustre des courbes de la quantité de charges mesurée au niveau de l'électrode de collecte de charges (en unité arbitraire), en fonction du temps (en µs).

La courbe 31 correspond au cas dans lequel le pixel est surmonté par une vallée d'une empreinte.

La courbe 32 correspond au cas dans lequel le pixel est surmonté par une crête d'une empreinte.

A l'instant t1=0, le chauffage par les première et deuxième résistances électriques 220A, 220B est activé. Ce chauffage reste activé jusqu'à t2=1600 µs.

Ici, le capteur est configuré pour que la cellule de calibration génère une quantité de charges permettant de mesurer une variation de charges positive ou négative, selon que le pixel est surmonté par une crête ou par une vallée d'une empreinte.

La courbe 31 passe de la valeur nulle à la valeur 0,25.

La courbe 32 passe de la valeur nulle à la valeur -0,15.

Le contraste correspondant est de 100%.

La figure 4 illustre une variante du capteur illustré en figure 2.

Cette variante ne diffère du capteur illustré en figure 2 qu'en ce que les pixels 400 ne comportent pas d'électrode(s) de référence distincte(s) d'un élément de chauffage.

En particulier, un même élément 420A, respectivement 420B, forme à la fois un élément de chauffage et une électrode de référence, pour la première respectivement deuxième cellule de détection thermique.

Cet élément est constitué d'une résistance électrique 420A, respectivement 420B, connectée à une source de potentiel constant pouvant prendre alternativement une valeur nulle et une valeur non nulle.

La résistance électrique 420A, 420B forme alors un élément de chauffage, activé lorsque ce potentiel prend une valeur non nulle.

Elle forme également une électrode de référence d'une première, respectivement deuxième capacité pyroélectrique 40A, 40B, puisqu'elle est connectée à une source de potentiel constant.

Le capteur est ainsi simplifié, puisque dans chaque cellule de calibration l'ensemble constitué d'un élément de chauffage, une couche d'isolation électrique, et une électrode de référence, est remplacé par cet unique élément 420A, respectivement 420B. Ainsi, la cellule de calibration 41A correspond directement à la première capacité pyroélectrique 40A, et la cellule de mesure 41B correspond directement à la deuxième capacité pyroélectrique 40B.

Cette variante tire avantage du fait qu'une électrode de référence et un élément de chauffage peuvent être tous deux constitués d'un même matériau.

Le capteur selon cette variante présente ici une couche de blindage 440B, qui s'étend entre la surface de contact 471 et la cellule de mesure 41B, pour protéger le capteur à l'égard des parasites électrostatiques, notamment autour de 50Hz. Cette couche de blindage 440B, conductrice d'électricité, est isolée électriquement de la deuxième résistance électrique 420B par une couche en matériau diélectrique 430B, présentant à la fois des propriétés d'isolant électrique et de conducteur thermique. Elle s'étend de préférence d'un seul tenant et sans ouverture au-dessus du substrat, en passant par l'ensemble des pixels du capteur.

Selon une autre variante, non représentée, le capteur selon l'invention ne présente pas de blindage électromagnétique. En effet, puisque la cellule de mesure et la cellule de calibration d'un même pixel sont soumises aux mêmes perturbations, et que l'on mesure l'équivalent d'un différentiel de charges, on peut s'attendre à ce que les charges générées par ces perturbations s'annulent, de sorte qu'un tel blindage n'est plus nécessaire. En pratique, cette annulation du bruit est difficilement atteignable. Le capteur peut comprendre alors des pixels non polarisés, pour mesurer et soustraire une perturbation commune à l'ensemble du capteur (bruits en mode commun de la matrice de pixels).

On décrit ensuite un deuxième mode de réalisation de l'invention, dans lequel la soustraction de charges n'est pas réalisée par addition de charges positives et négatives, au niveau d'une électrode commune, mais par mesure différentielle à partir de signaux collectés par deux électrodes distinctes.

Ce deuxième mode de réalisation est particulièrement avantageux sur un substrat en silicium, sur lequel il est plus aisé de multiplier un nombre de connexions électriques.

Ce mode de réalisation ne sera décrit que pour ses différences relativement au premier mode de réalisation.

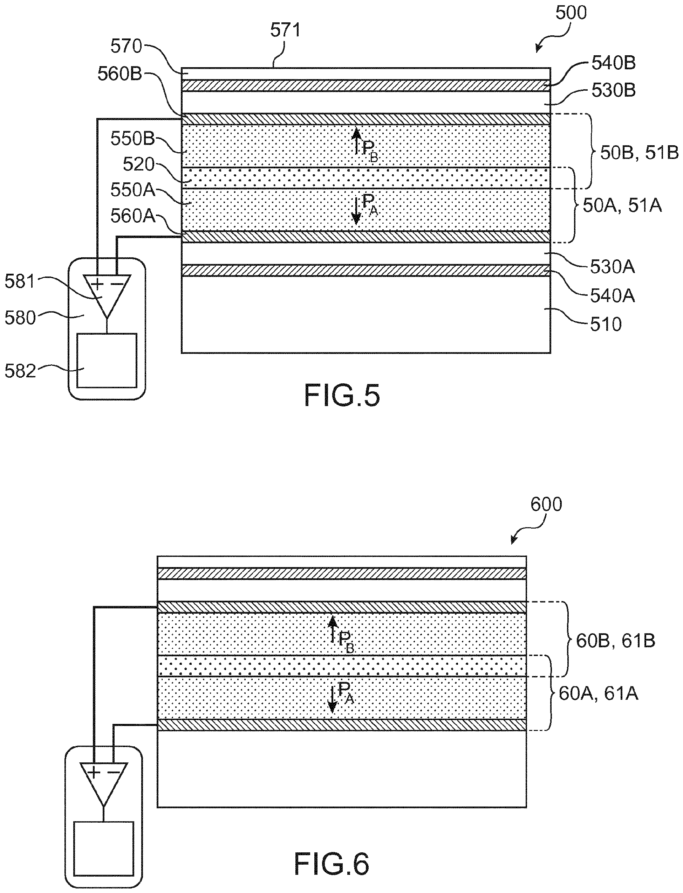

Selon une première variante, illustrée à la figure 5, chaque pixel 500 du capteur comprend, superposés dans cet ordre au-dessus du substrat 510 :
- une première couche conductrice 540A, connectée à une source de potentiel constant, par exemple à la masse ;
- une première couche en matériau diélectrique 530A ;
- une première électrode 560A de collecte de charges ;
- une première portion 550A comprenant un matériau pyroélectrique ;
- une électrode centrale 520, connectée à une source de potentiel constant, pouvant prendre alternativement une valeur nulle et une valeur non nulle, formant à la fois une électrode de référence, et un élément de chauffage activé lorsque ce potentiel prend une valeur non nulle ;
- une deuxième portion 550B comprenant un matériau pyroélectrique ;
- une deuxième électrode 560B de collecte de charges ;
- une deuxième couche en matériau diélectrique 530B ;
- une deuxième couche conductrice 540B, connectée à une source de potentiel constant, par exemple à la masse, formant un blindage électromagnétique pour protéger le capteur ; et
- une couche de protection 570, optionnelle, telle que décrite ci-avant.

Afin d'optimiser le blindage, la deuxième couche conductrice 540B s'étend d'un seul tenant et sans ouverture au-dessus du substrat, en passant par l'ensemble des pixels du capteur.

Comme en figure 4, cet exemple tire avantage du fait qu'une électrode de référence et un élément de chauffage peuvent être tous deux constitués d'un même matériau. Ici, c'est l'électrode centrale 520 qui forme à la fois une électrode de référence et un élément de chauffage.

La première portion comprenant un matériau pyroélectrique 550A s'étend ici en contact physique direct, côté inférieur avec la première électrode 560A de collecte de charges, et côté supérieur avec l'électrode centrale 520.

Comme précédemment, une ou plusieurs couches intercalaires conductrices d'électricité peuvent s'insérer entre la première portion 550A et l'électrode centrale 520, et/ou entre la première portion 550A et la première électrode 560A de collecte de charges. On définit une première structure, comportant au moins la première portion 550A, et, le cas échéant, cette ou ces couche(s) intercalaire(s).

Ladite première structure, la première électrode 560A de collecte de charges et l'électrode centrale 520, forment ensemble une première capacité pyroélectrique 50A, située du côté du substrat.

De la même façon, la deuxième portion 550B s'étend ici en contact physique direct, côté inférieur avec l'électrode centrale 520, et côté supérieur avec la deuxième électrode 560B de collecte de charges.

Le cas échéant, une ou plusieurs couches intercalaires conductrices d'électricité peuvent s'insérer entre la deuxième portion 550B et l'électrode centrale 520, et/ou entre la deuxième portion 550B et la deuxième électrode 560B de collecte de charges. On définit une deuxième structure, comportant au moins la deuxième portion 550B, et, le cas échéant, cette ou ces couche(s) intercalaire(s).

Ladite deuxième structure, l'électrode centrale 520 et la deuxième électrode 560B de collecte de charges, forment ensemble une deuxième capacité pyroélectrique 50B, située du côté de la surface de contact 571.

Les deux capacités pyroélectriques 50A, 50B peuvent être chauffées par l'électrode centrale 520, pour la mise en œuvre d'une détection de type actif. Par construction, le chauffage est donc symétrique dans les deux capacités pyroélectriques 50A, 50B.

Comme à la figure 4, une première cellule de détection thermique 51A, comportant la première capacité pyroélectrique 50A et son élément de chauffage 520, correspond directement à la première capacité pyroélectrique 50A.

De la même façon, une deuxième cellule de détection thermique 51B, comportant la deuxième capacité pyroélectrique 50B et son élément de chauffage 520, correspond directement à ladite deuxième capacité pyroélectrique 50B.

Comme précédemment, l'agencement des deux cellules 51A, 51B présente une symétrie, relativement à un plan parallèle à la surface de contact du capteur, passant ici par l'électrode centrale 520.

La première cellule 51A, située du côté du substrat, forme une cellule de calibration telle que décrite ci-avant.

La deuxième cellule de détection thermique 51B, située du côté de la surface de contact 571, forme une cellule de mesure telle que décrite ci-avant.

Comme précédemment, il s'agit de soustraire des charges pyroélectriques de calibration, générées dans la cellule de calibration 51A, à des charges pyroélectrique de mesure, générées dans la cellule de mesure 51B (ou inversement).

Ici, les charges de calibration sont collectées par la première électrode 560A de collecte de charges, tandis que les charges de mesure sont collectées par la deuxième électrode 560B de collecte de charges.

Comme précédemment, les axes de polarisation P_{A} et P_{B} des portions 550A, 550B comprenant un matériau pyroélectrique, sont orientés parallèles à l'axe z. En revanche, ces axes sont orientés ici dans des sens opposés (tous deux vers l'électrode centrale, ou tous deux vers l'électrode de collecte de charges correspondante).

Les charges de mesure et les charges de calibration présentent donc le même signe (positif ou négatif). On réalise donc la soustraction de charges au niveau d'un dispositif électronique 580, relié aux deux électrodes de collecte de charges 560A, 560B.

Le dispositif électronique 580 comporte ici un amplificateur différentiel 581, connecté en entrée d'une part à la première électrode 560A de collecte de charges, d'autre part à la deuxième électrode 560B de collecte de charges, et fournissant en sortie un signal différentiel correspondant à la différence entre les deux signaux d'entrée. L'amplificateur différentiel 581 est connecté ensuite à un dispositif électronique 582, fournissant en sortie une tension, ou directement une valeur numérique s'il contient un convertisseur analogique-numérique, qui dépend de la différence entre les charges de mesure et les charges de calibration (ou inversement).

Comme précédemment, un élément conducteur d'électricité situé entre les deux portions en matériau pyroélectrique, ici l'électrode commune 520, peut présenter des propriétés d'isolant thermique (notamment être en PEDOT:PSS).

On remarque qu'il n'est pas nécessaire que le pixel présente un blindage électromagnétique, du côté du substrat. Par conséquent, la première couche conductrice 540A, sert simplement à améliorer la symétrie du pixel.

Les inventeurs ont montré qu'il est possible de s'affranchir de cette première couche conductrice 540A (et de la première couche en matériau diélectrique 530A), le défaut de symétrie engendré étant négligeable, ou pouvant être compensé par d'autres moyens. (voir ci-après). Cette variante est illustrée en figure 6, sur laquelle les références numériques correspondent à celles de la figure 5, le premier chiffre étant remplacé par un 6.

Selon une autre variante, non représentée, le capteur selon l'invention ne présente pas la deuxième couche conductrice 540B, formant une couche de blindage électromagnétique. En effet, on peut s'attendre à ce que les sources de bruit perturbent de la même façon les deux capacités pyroélectriques 60A, 60B (bruits injectés par le système de chauffage notamment). Dans ce cas, l'effet du bruit est annulé par la mesure différentielle, et on peut s'affranchir d'une couche de blindage électromagnétique.

Selon une autre variante, non représentée, la cellule de mesure et la cellule de calibration comportent chacune une résistance distincte formant à la fois électrode de référence et élément de chauffage, les deux résistances distinctes étant disposées entre les deux portions comprenant un matériau pyroélectrique.

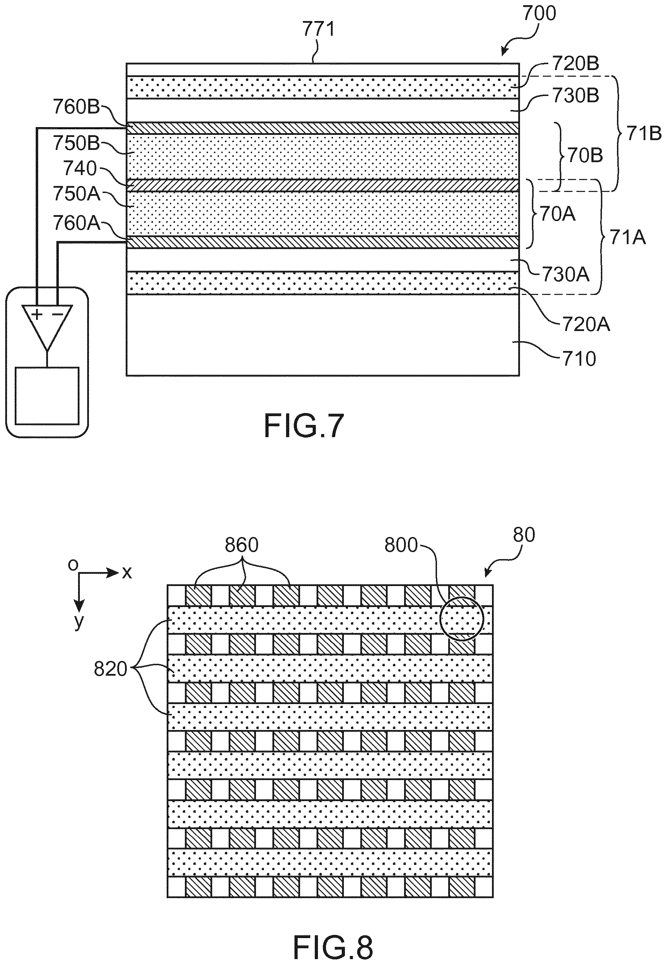

Selon une autre variante, illustrée en figure 7, le chauffage n'est pas mis en œuvre par un élément central disposé entre les deux portions comportant un matériau pyroélectrique, mais par deux éléments distincts disposés symétriquement de part et d'autre de ces deux portions.

En particulier, le pixel 700 comporte :
- une première résistance électrique 720A, située entre le substrat 710 et la première électrode 760A de collecte de charges, adaptée à être connectée à une source de courant ou de tension, et formant un élément de chauffage disposé du côté du substrat 710 ; et
- une deuxième résistance électrique 720B, située entre la deuxième électrode 760B de collecte de charges et la surface de contact 771, adaptée à être connectée à une source de courant ou de tension, et formant un élément de chauffage disposé du côté de la surface de contact 771.

Chaque résistance électrique 720A, 720B, est séparée de l'électrode de collecte de charges voisine 760A, respectivement 760B, par une couche en matériau diélectrique 730A, respectivement 730B.

Là encore, on peut définir une première capacité pyroélectrique 70A, et une deuxième capacité pyroélectrique 70B, partageant ici une électrode centrale, référencée 740.

La première capacité pyroélectrique 70A forme, avec la première résistance électrique 720A et la première couche en matériau diélectrique 730A, une cellule de calibration 71A.

La deuxième capacité pyroélectrique 70B forme, avec la deuxième résistance électrique 720B et la deuxième couche en matériau diélectrique 730B, une cellule de mesure 71B.

Selon cette variante, l'électrode centrale, 740, sert uniquement d'électrode de référence commune pour les deux capacités pyroélectriques 70A, 70B.

Les deux capacités pyroélectriques 70A, 70B étant reliées à un même amplificateur différentiel, et agencées symétriques l'une au-dessus de l'autre, on peut s'affranchir de cette électrode centrale (variante non représentée). Chaque pixel est alors dépourvu d'une électrode de référence, située entre la première et la deuxième structures.

Dans ce cas, la première et la deuxième portions 750A, respectivement 750B, ne sont pas séparées l'une de l'autre par une couche conductrice connectée à une source de potentiel constant. Elles peuvent s'étendre en contact physique direct l'une avec l'autre, ou de part et d'autre d'une couche de colle conductrice d'électricité mais non connectée pendant le fonctionnement.

La figure 8 illustre de manière schématique, selon une vue de dessus, un agencement avantageux des électrodes de collecte de charges et des éléments de chauffage, dans un capteur 80 selon l'invention.

Selon cet agencement, les électrodes 860 de collecte de charges s'étendent selon une série de bandes parallèles entre elles, lorsque les pixels comportent chacun une électrode commune de collecte de charges (premier mode de réalisation), ou selon deux séries de bandes parallèles entre elles, lorsque les pixels comportent chacun deux électrodes de collecte de charge (deuxième mode de réalisation). Lorsque les électrodes 860 s'étendent selon deux séries de bandes, les bandes sont superposées deux à deux au-dessus du substrat.

Chaque série de bandes s'étend dans un plan parallèle au plan de la surface de contact du capteur.

De la même façon, les éléments de chauffage 820 s'étendent selon deux séries de bandes parallèles entre elles, lorsque les pixels comportent chacun deux éléments de chauffage (figures 2, 4, 7), ou selon une série de bandes parallèles entre elles, lorsque les pixels comportent chacun un élément de chauffage commun (figures 5 et 6). Lorsque les éléments de chauffage 820 s'étendent selon deux séries de bandes, les bandes sont superposées deux à deux au-dessus du substrat. En d'autres termes, les éléments de chauffage en forme de bande sont alors disposés deux à deux face à face. Chaque élément de chauffage en forme de bande peut être nommé simplement « bande chauffante ».

Chaque série de bandes s'étend dans un plan parallèle au plan de la surface de contact du capteur.

Les éléments de chauffage 820 et les électrodes 860 s'étendent selon des directions croisées, de préférence orthogonales.

Chaque pixel 800 correspond à une région d'intersection, selon une vue de dessus, entre une électrode 860 (ou deux électrodes superposées), et un élément de chauffage 820 (ou deux éléments superposés). On peut identifier précisément le pixel concerné, lors de la lecture d'une électrode de collecte de charges, sachant quel est l'élément de chauffage qui est activé.

La matrice de pixels forme ainsi une matrice passive, présentant l'avantage de nécessiter un nombre plus restreint de connexions électriques. Cet avantage présente un intérêt particulier dans le deuxième mode de réalisation, où chaque pixel comporte deux électrodes de collecte de charge.

Dans ce cas, chaque bande chauffante peut être connectée en série ou en parallèle à la bande chauffante située en face, de sorte que deux bandes chauffantes se faisant face sont connectées à une même source de courant ou de tension.

Elles peuvent en outre présenter deux à deux les mêmes dimensions et la même composition, de sortent qu'elles forment la même résistance et fournissent un même chauffage.

L'invention n'est pas limitée à cet agencement. La matrice de pixel du capteur selon l'invention peut, par exemple, former une matrice active, dans laquelle les électrodes de collecte de charges de chaque pixel sont distinctes les unes des autres, et connectées individuellement à des dispositifs électroniques de mesure.

Afin d'optimiser la calibration mise en œuvre, différentes solutions d'ajustement peuvent être mises en œuvre afin de soustraire la quantité souhaitée de charges de calibration.

En particulier, on ajuste au moins un paramètre relatif à une région supérieure du pixel, incluant la cellule de mesure, par rapport au paramètre correspondant sur une région inférieure du pixel, incluant la cellule de calibration, de manière à soustraire la quantité souhaitée de charges de calibration.

Cet ajustement peut être réalisé par un chauffage différencié des deux portions comprenant un matériau pyroélectrique.

Un tel ajustement est adapté, lorsque la cellule de mesure et la cellule de calibration présentent des éléments de chauffage distincts l'un de l'autre.

On ajuste par exemple la résistance électrique de l'élément de chauffage associé à l'une cellule, relativement à la résistance électrique de l'élément de chauffage associé à l'autre cellule.

Ces deux éléments de chauffage peuvent alors présenter des formes différentes (par exemple des épaisseurs et/ou des longueurs différentes).

Ils peuvent néanmoins être parcourus par un même courant.

Par exemple, le capteur selon l'invention peut comprendre des bandes chauffantes superposées deux à deux (voir figure 8), chaque bande chauffante et la bande chauffante lui faisant face étant connectées ensemble en série ou en parallèle, et présentant des dimensions différentes l'une de l'autre.

En particulier, chaque bande chauffante peut présenter une forme et des dimensions différentes de celles de la bande chauffante lui faisant face, de sorte que la différence entre des charges générées par la première portion en matériau pyroélectrique et des charges générées par la deuxième portion en matériau pyroélectrique d'un même pixel est nulle, lorsque le capteur est en contact physique direct avec de l'air, du côté opposé au substrat.

Un tel ajustement présente cependant l'inconvénient d'être statique.

En variante, un ajustement par le chauffage peut être obtenu en injectant différentes valeurs de courant ou de tension dans les deux éléments de chauffage d'un même pixel.

Un tel ajustement peut être mis en œuvre en temps réel, et adapté à des conditions réelles de fonctionnement.

Il permet en outre de réduire une consommation électrique du capteur, en minimisant des pertes thermiques vers le substrat, ces faibles pertes thermiques étant compensées par un chauffage plus faible côté substrat que côté surface de contact.

Par exemple, le capteur selon l'invention peut comprendre des bandes chauffantes superposées deux à deux (voir figure 8), chaque bande chauffante et la bande chauffante lui faisant face n'étant pas connectées ensemble, de façon à pouvoir être parcourues par différentes valeurs de courant.

En particulier, le courant injecté dans une bande chauffante et le courant injecté dans la bande chauffante lui faisant face, peuvent être adaptés de sorte que la différence entre des charges générées par la première portion en matériau pyroélectrique et des charges générées par la deuxième portion en matériau pyroélectrique d'un même pixel est nulle, lorsque le capteur est en contact physique direct avec de l'air, du côté opposé au substrat, et pour un temps d'intégration prédéterminée (durée associée aux variations de température considérées).

L'ajustement peut également être réalisé au niveau des deux portions comportant un matériau pyroélectrique.

En particulier, on peut ajuster :
- le coefficient pyroélectrique de l'une portion comprenant un matériau pyroélectrique, relativement au coefficient pyroélectrique l'autre portion comprenant un matériau pyroélectrique (en ne les polarisant pas exactement de la même manière, ou en utilisant deux matériaux différents) ; et/ou
- l'épaisseur de l'une portion comprenant un matériau pyroélectrique, relativement à l'épaisseur de l'autre portion comprenant un matériau pyroélectrique (sachant que les charges générées par chacune desdites portions ne dépendent pas de l'épaisseur, mais l'élévation de température sera différente).

Enfin, l'ajustement peut également être réalisé par un choix adapté de matériaux et d'épaisseurs pour les différentes parties constituant un pixel du capteur.

On ajuste en particulier les caractéristiques thermiques des différentes couches composant un pixel du capteur selon l'invention, notamment la conduction et la capacité thermiques.

Ces grandeurs dépendent de la nature des matériaux et de leurs épaisseurs.

Les couches constituant la cellule de mesure et celles constituant la cellule de calibration étant de préférence symétriques entre elles, un tel ajustement est mis en œuvre de préférence par un ajustement des caractéristiques thermiques du substrat.

Par exemple, le capteur peut être configuré pour que des transferts thermiques entre la première portion comportant un matériau pyroélectrique et le substrat, correspondent sensiblement à des transferts thermiques entre la deuxième portion comportant un matériau pyroélectrique et une crête d'une empreinte (lorsque l'empreinte est appuyée contre la surface de contact du capteur). Les caractéristiques thermiques de la peau peuvent être approximées par celles de l'eau. On choisit alors un substrat présentant sensiblement la même conduction thermique que l'eau (en supposant que l'effet thermique de la couche de protection est négligeable).

Selon une autre variante, le capteur selon l'invention peut être configuré pour que des transferts thermiques entre la première portion comportant un matériau pyroélectrique et le substrat, soient inférieurs à des transferts thermiques entre la deuxième portion comportant un matériau pyroélectrique et une crête d'une empreinte, et supérieurs à des transferts thermiques entre la deuxième portion comportant un matériau pyroélectrique et une vallée d'une empreinte (lorsque l'empreinte est appuyée contre la surface de contact du capteur).

On choisit alors un substrat présentant une conduction thermique comprise entre celle de l'eau et celle de l'air.

Cet ajustement par les caractéristiques thermiques peut également prendre en compte les caractéristiques thermiques de couches intermédiaires situées entre la cellule de mesure et la surface de contact et/ou entre la cellule de calibration et le substrat, par exemple les caractéristiques thermiques d'une couche de protection.

De préférence, un tel ajustement prend également en considération les caractéristiques thermiques d'un support sur lequel repose le substrat, en utilisation.

Les différentes solutions d'ajustement mentionnées ci-dessus peuvent être combinées entre elles.

On remarque que l'ajustement se traduit par une légère perte de symétrie entre une cellule de mesure et la cellule de calibration correspondante (au niveau du chauffage, ou au niveau de la structure du capteur).

L'homme du métier saura, notamment par des simulations, dimensionner un capteur selon l'invention offrant la calibration voulue.

En particulier, la composition et l'épaisseur des différents éléments constitutifs d'un pixel du capteur peuvent être adaptées de sorte que la différence entre des charges générées par la première portion en matériau pyroélectrique et des charges générées par la deuxième portion en matériau pyroélectrique d'un même pixel soit nulle, lorsque le capteur est en contact physique direct avec de l'air, du côté opposé au substrat.

Dans chacun des modes de réalisation, on retrouve une certaine symétrie entre une région supérieure (côté surface de contact) et une région inférieure (côté substrat) du pixel. En outre, l'agencement du pixel permet un chauffage sensiblement symétrique de ces deux régions, par un élément de chauffage central, ou par deux éléments de chauffage.

L'invention n'est pas limitée aux exemples décrits, de nombreuses autres variantes pouvant être mises en œuvre, permettant de définir deux cellules pyroélectriques superposées mettant chacune en œuvre une détection thermique de type actif.

On remarque que sauf exception, chaque pixel du capteur selon l'invention comporte deux capacités pyroélectriques superposées, chacune comportant une portion comprenant un matériau pyroélectrique, une électrode de collecte de charge, et une électrode de référence. Les deux capacités pyroélectriques peuvent partager une même électrode de collecte de charge ou une même électrode de référence.

Par exemple, dans chaque pixel, une couche d'isolation thermique, électriquement conductrice, peut être située entre la première et la deuxième portions comprenant un matériau pyroélectrique.

En outre, l'idée à la base de l'invention peut être adaptée à une capture d'image à l'aide d'une matrice de thermistances (élément résistif présentant une résistance électrique qui varie en fonction de la température). Chaque pixel du capteur présente alors deux thermistances superposées verticalement, l'une dédiée à la mesure et l'autre à la calibration. Ces deux thermistances sont séparées de préférence par une couche isolante thermiquement. On soustrait ensuite les signaux mesurés respectivement sur la thermistance de mesure et sur la thermistance de calibration. Pour cela, on peut par exemple polariser lesdites deux thermistances, montées en série, avec un potentiel connu, et lire un potentiel entre ces deux thermistances. Selon une variante avantageuse, on mesure une différence entre ce potentiel entre les deux thermistances, et un potentiel entre deux thermistances de référence.

## Revendications

1. Capteur (80) de motifs thermiques, de type capteur pyroélectrique, comportant une surface de contact (271 ; 571; 771) pour y appliquer un objet à imager, en particulier une empreinte papillaire, et une pluralité de pixels (200; 400; 500; 600 ; 700 ; 800) répartis entre un substrat (210; 510 ; 710 ; 910) et ladite surface de contact (271 ; 571 ; 771), **caractérisé en ce que** chaque pixel comprend :
- au moins une électrode de collecte de charges (260 ; 460 ; 560A ; 760A ; 860) ;
- une première structure, comportant une première portion (250A; 450A; 550A; 750A) comprenant un matériau pyroélectrique, ladite première structure étant en contact physique direct avec l'une parmi l'au moins une électrode de collecte de charges (260 ; 460 ; 560A ; 760A ; 860) ;
- une deuxième structure, comportant une deuxième portion (250B ; 450B; 550B ; 750B) comprenant un matériau pyroélectrique, ladite deuxième structure étant en contact physique direct avec l'une parmi l'au moins une électrode de collecte de charges (260 ; 460 ; 560B ; 760B ; 860), avec la première structure, la deuxième structure, et l'au moins une électrode de collecte de charge superposées au-dessus du substrat et agencées de sorte que chaque pixel comporte deux capacités pyroélectriques superposées, où chaque capacité pyroélectrique comporte, au moins, l'une portion comprenant un matériau pyroélectrique et l'une électrode de collecte de charge, et avec les deux capacités pyroélectriques qui partagent une même électrode de collecte de charge ou une même électrode de référence ;
- au moins un élément de chauffage (220A, 220B ; 420A, 420B ; 520 ; 720A, 720B ; 820), pour chauffer la première et la deuxième portions comprenant un matériau pyroélectrique ; et
- un dispositif électronique (280 ; 580) connecté à l'au moins une électrode de collecte de charges, et configuré pour mesurer une différence entre des charges générées par l'une parmi la première et la deuxième portions comprenant un matériau pyroélectrique, et des charges générées par l'autre parmi la première et la deuxième portions comprenant un matériau pyroélectrique.

2. Capteur (80) selon la revendication 1, **caractérisé en ce que** la première portion (250A ; 450A ; 550A ; 750A) comprenant un matériau pyroélectrique et la deuxième portion (250B ; 450B ; 550B ; 750B) comprenant un matériau pyroélectrique présentent une même épaisseur et une même composition chimique.

3. Capteur (80) selon la revendication 1 ou 2, **caractérisé en ce que** chaque pixel comporte deux capacités pyroélectriques superposées, chacune comportant l'une portion comprenant un matériau pyroélectrique, l'une électrode de collecte de charge, et une électrode de référence, avec les deux capacités pyroélectriques qui partagent une même électrode de collecte de charge ou une même électrode de référence.

4. Capteur (80) selon l'une quelconque des revendications 1 à 3, **caractérisé en ce que** la première et la deuxième structures sont chacune en contact physique direct avec la même électrode de collecte de charges (260; 460), dite électrode commune, située entre la première et la deuxième structures.

5. Capteur (80) selon la revendication 4, **caractérisé en ce que** la première et la deuxième portions comprenant un matériau pyroélectrique présentent chacune une polarisation respective (P_{A}, P_{B}), ces polarisations étant orientées dans la même direction et le même sens.

6. Capteur (80) selon la revendication 4 ou 5, **caractérisé en ce que** ledit dispositif électronique (280) est configuré pour mesurer des charges collectées par ladite électrode commune (260 ; 460).

7. Capteur (80) selon l'une quelconque des revendications 4 à 6, **caractérisé en ce que** chaque pixel (200 ; 400) comporte également deux électrodes de référence (240A, 240B ; 420A, 420B), situées de part et d'autre d'un empilement comportant la première structure, l'électrode commune, et la deuxième structure.

8. Capteur (80) selon l'une quelconque des revendications 1 à 3, **caractérisé en ce que** la première structure et la deuxième structure sont chacune en contact physique direct avec deux électrodes respectives de collecte de charges (560A, 560B ; 760A, 760B), l'une (560A; 760A) étant située d'un côté d'un empilement comprenant la première et la deuxième structures, et l'autre (560B ; 760B) étant située de l'autre côté de cet empilement.

9. Capteur (80) selon la revendication 8, **caractérisé en ce que** la première et la deuxième portions comprenant un matériau pyroélectrique présentent chacune une polarisation respective (P_{A}, P_{B}), ces polarisations étant orientées dans des sens opposés.

10. Capteur (80) selon la revendication 8 ou 9, **caractérisé en ce que** ledit dispositif électronique (580) comprend un amplificateur différentiel (581), connecté en entrée à l'une et à l'autre des deux électrodes de collecte de charges.

11. Capteur (80) selon l'une quelconque des revendications 8 à 10, **caractérisé en ce que** chaque pixel (500 ; 600) comporte également une électrode de référence (520), située entre la première et la deuxième structures.

12. Capteur (80) selon l'une quelconque des revendications 8 à 11, **caractérisé en ce que** l'au moins un élément de chauffage comprend un élément de chauffage (520) en matériau électriquement conducteur, situé entre la première et la deuxième structures.

13. Capteur (80) selon l'une quelconque des revendications 1 à 11, **caractérisé en ce que** l'au moins un élément de chauffage comprend deux éléments de chauffage (220A, 220B ; 420A, 420B ; 720A, 720B) en matériau électriquement conducteur, situés de part et d'autre d'un empilement comportant la première et la deuxième structures.

14. Capteur (80) selon la revendication 13, **caractérisé en ce que** les éléments de chauffage (220A, 220B ; 420A, 420B ; 720A, 720B) des différents pixels forment ensemble des bandes chauffantes, réparties de part et d'autre dudit empilement, et disposées deux à deux face à face.

15. Capteur (80) selon l'une quelconque des revendications 1 à 14, **caractérisé en ce que** chaque pixel comporte également une portion de colle (93), située entre les première et deuxième structures.

## Patentansprüche

1. Wärmemustersensor (80) vom Typ pyroelektrischer Sensor mit einer Kontaktfläche (271; 571; 771), um darauf ein abzubildendes Objekt, insbesondere einen Papillarabdruck, aufzubringen, und einer Vielzahl von Pixeln (200; 400; 500; 600; 700; 800), die zwischen einem Substrat (210; 510; 710; 910) und der genannten Kontaktfläche (271; 571; 771) verteilt sind, **dadurch gekennzeichnet, dass** jedes Pixel enthält:
- zumindest eine Ladungssammelelektrode (260; 460; 560A; 760A, 860);
- eine erste Struktur, enthaltend einen ersten Abschnitt (250A; 450A; 550A; 750A), der ein pyroelektrisches Material enthält, wobei die erste Struktur in direkter Berührung mit einer der zumindest einen Ladungssammelelektrode (260; 460; 560A; 760A; 860) steht,
- eine zweite Struktur, enthaltend einen zweiten Abschnitt (250B; 450B; 550B; 750B), der ein pyroelektrisches Material enthält, wobei die zweite Struktur in direkter Berührung mit einer der zumindest einen Ladungssammelelektrode (260; 460; 560B; 760B; 860) steht, wobei die erste Struktur, die zweite Struktur und die zumindest eine Ladungssammelelektrode über dem Substrat übereinandergeordnet und so angeordnet sind, dass jedes Pixel zwei übereinandergeordnete pyroelektrische Kapazitäten umfasst, wobei jede pyroelektrische Kapazität zumindest den einen pyroelektrisches Material enthaltenden Abschnitt und die eine Ladungssammelelektrode umfasst, und wobei sich die beiden pyroelektrischen Kapazitäten dieselbe Ladungssammelelektrode bzw. dieselbe Referenzelektrode teilen;
- zumindest ein Heizelement (220A, 220B; 420A, 420B; 520; 720A, 720B; 820) zum Erwärmen des ersten und des zweiten pyroelektrisches Material enthaltenden Abschnitts; und
- eine elektronische Vorrichtung (280; 580), die mit der zumindest einen Ladungssammelelektrode verbunden und dazu ausgelegt ist, eine Differenz zwischen Ladungen zu messen, die von einem aus erstem und zweitem pyroelektrisches Material enthaltenden Abschnitt erzeugt werden, sowie Ladungen, die von dem anderen aus erstem und zweitem pyroelektrisches Material enthaltenden Abschnitt erzeugt werden.

2. Sensor (80) nach Anspruch 1,
**dadurch gekennzeichnet, dass**
der erste pyroelektrisches Material enthaltende Abschnitt (250A; 450A; 550A; 750A) und der zweite pyroelektrisches Material enthaltende Abschnitt (250B; 450B; 550B, 750B) die gleiche Dicke und die gleiche chemische Zusammensetzung aufweisen.

3. Sensor (80) nach Anspruch 1 oder 2,
**dadurch gekennzeichnet, dass**
jedes Pixel zwei übereinandergeordnet pyroelektrische Kapazitäten enthält, die jeweils die einen pyroelektrisches Material enthaltenden Abschnitt, die eine Ladungssammelelektrode und eine Referenzelektrode enthalten, wobei die beiden pyroelektrischen Kapazitäten dieselbe Ladungssammelektrode bzw. dieselbe Referenzelektrode teilen.

4. Sensor (80) nach einem der Ansprüche 1 bis 3,
**dadurch gekennzeichnet, dass**
die erste und die zweite Struktur jeweils in direkter Berührung mit derselben Ladungssammelelektrode (260; 460), gemeinsame Elektrode genannt, stehen, die zwischen der ersten und der zweiten Struktur liegt.

5. Sensor (80) nach Anspruch 4,
**dadurch gekennzeichnet, dass**
der erste und der zweite pyroelektrisches Material enthaltende Abschnitt jeweils eine jeweilige Polarisation (P_{A}, P_{B}) haben, wobei diese Polarisationen in der gleichen Ausrichtung und in der gleichen Richtung ausgerichtet sind.

6. Sensor (80) nach Anspruch 4 oder 5,
**dadurch gekennzeichnet, dass**
die elektronische Vorrichtung (280) dazu ausgelegt ist, von der gemeinsamen Elektrode (260; 460) gesammelte Ladungen zu messen.

7. Sensor (80) nach einem der Ansprüche 4 bis 6,
**dadurch gekennzeichnet, dass**
jedes Pixel (200; 400) auch zwei Referenzelektroden (240A, 240B; 420A, 420B) enthält, die beiderseits einer Stapelung liegen, welche die erste Struktur, die gemeinsame Elektrode und die zweite Struktur umfasst.

8. Sensor (80) nach einem der Ansprüche 1 bis 3,
**dadurch gekennzeichnet, dass**
die erste Struktur und die zweite Struktur jeweils in direkter Berührung mit zwei jeweiligen Ladungssammelelektroden (560A, 560B; 760A, 760B) stehen, wobei die eine (560A; 760A) auf einer Seite einer die erste und die zweite Struktur umfassenden Stapelung liegt und die andere (560B; 760B) auf der anderen Seite dieser Stapelung liegt.

9. Sensor (80) nach Anspruch 8,
**dadurch gekennzeichnet, dass**
der erste und der zweite pyroelektrisches Material enthaltende Abschnitt jeweils eine jeweilige Polarisation (P_{A}, P_{B}) aufweisen, wobei diese Polarisierungen in entgegengesetzten Richtungen ausgerichtet sind.

10. Sensor (80) nach Anspruch 8 oder 9,
**dadurch gekennzeichnet, dass**
die elektronische Vorrichtung (580) einen Differenzverstärker (581) enthält, der am Eingang mit der einen und der anderen der beiden Ladungssammelelektroden verbunden ist.

11. Sensor (80) nach einem der Ansprüche 8 bis 10,
**dadurch gekennzeichnet, dass**
jedes Pixel (500; 600) auch eine Referenzelektrode (520) enthält, die zwischen der ersten und der zweiten Struktur liegt.

12. Sensor (80) nach einem der Ansprüche 8 bis 11,
**dadurch gekennzeichnet, dass**
das zumindest eine Heizelement ein Heizelement (520) aus elektrisch leitfähigem Material enthält, das zwischen der ersten und der zweiten Struktur liegt.

13. Sensor (80) nach einem der Ansprüche 1 bis 11,
**dadurch gekennzeichnet, dass**
das zumindest eine Heizelement zwei Heizelemente (220A, 220B; 420A, 420B; 720A, 720B) aus elektrisch leitfähigem Material enthält, die beiderseits einer Stapelung liegen, welche die erste und die zweite Struktur umfasst.

14. Sensor (80) nach Anspruch 13,
**dadurch gekennzeichnet, dass**
die Heizelemente (220A, 220B; 420A, 420B; 720A, 720B) der verschiedenen Pixel zusammen Heizbänder bilden, die auf beiden Seiten der Stapelung verteilt und paarweise einander gegenüberliegend angeordnet sind.

15. Sensor (80) nach einem der Ansprüche 1 bis 14,
**dadurch gekennzeichnet, dass**
jedes Pixel auch einen Klebeabschnitt (93) enthält, der sich zwischen der ersten und zweiten Struktur befindet.

## Claims

1. A sensor (80) for thermal patterns, of the pyroelectric sensor type, including a contact surface (271; 571; 771) to apply an object to be imaged thereto, in particular a fingerprint, and a plurality of pixels (200; 400; 500; 600; 700; 800) distributed between a substrate (210; 510; 710; 910) and said contact surface (271; 571; 771), **characterised in that** each pixel comprises:
- at least one charge collecting electrode (260; 460; 560A; 760A; 860);
- a first structure, including a first portion (250A; 450A; 550A; 750A) comprising a pyroelectric material, said first structure being in direct physical contact with one from the at least one charge collecting electrode (260; 460; 560A; 760A; 860);
- a second structure, including a second portion (250B; 450B; 550B; 750B) comprising a pyroelectric material, said second structure being in direct physical contact with one from the at least one charge collecting electrode (260; 460; 560A; 760A; 860), with the first structure, the second structure, and the at least one charge collecting electrode that are superimposed above the substrate and arranged so that each pixel includes two superimposed pyroelectric capacitors, where each pyroelectric capacitor includes, at least, the one portion comprising a pyroelectric material and the one charge collecting electrode, and with both pyroelectric capacitors sharing a same charge collecting electrode or a same reference electrode;
- at least one heating element (220A, 220B; 420A, 420B; 520; 720A, 720B; 820), to heat the first and second portions comprising a pyroelectric material; and
- an electronic device (280; 580) connected to the at least one charge collecting electrode, and configured to measure a difference between charges generated by one from the first and second portions comprising a pyroelectric material, and charges generated by the other from the first and second portions comprising a pyroelectric material.

2. The sensor (80) according to claim 1, **characterised in that** the first portion (250A; 450A; 550A; 750A) comprising a pyroelectric material and the second portion (250B; 450B; 550B; 750B) comprising a pyroelectric material have a same thickness and a same chemical composition.

3. The sensor (80) according to claim 1 or 2, **characterised in that** each pixel includes two superimposed pyroelectric capacitors, each including the one portion comprising a pyroelectric material, the one charge collecting electrode, and a reference electrode, with both pyroelectric capacitors sharing a same charge collecting electrode or a same reference electrode.

4. The sensor (80) according to any of claims 1 to 3, **characterised in that** the first and the second structures are each in direct physical contact with the same charge collecting electrode (260; 460), referred to as the common electrode, located between the first and second structures.

5. The sensor (80) according to claim 4, **characterised in that** the first and second portions comprising a pyroelectric material each have a respective bias (P_{A}, P_{B}), these biases being oriented in the same direction and the same sense.

6. The sensor (80) according to claim 4 or 5, **characterised in that** said electronic device (280) is configured to measure charges collected by said common electrode (260; 460).

7. The sensor (80) according to any of claims 4 to 6, **characterised in that** each pixel (200; 400) also includes two reference electrodes (240A, 240B; 420A, 420B), located on either side of a stack including the first structure, the common electrode, and the second structure.

8. The sensor (80) according to any of claims 1 to 3, **characterised in that** the first structure and the second structure are each in direct physical contact with two respective charge collecting electrodes (560A, 560B; 760A, 760B), one (560A; 760A) being located on a side of a stack comprising the first and second structures, and the other (560B; 760B) being located on the other side of this stack.

9. The sensor (80) according to claim 8, **characterised in that** the first and second portions comprising a pyroelectric material each have a respective bias (P_{A}, P_{B}), these biases being oriented in opposite senses.

10. The sensor (80) according to claim 8 or 9, **characterised in that** said electronic device (580) comprises a differential amplifier (581), connected at the input to each of both charge collecting electrodes.

11. The sensor (80) according to any of claims 8 to 10, **characterised in that** each pixel (500; 600) also includes a reference electrode (520), located between the first and second structures.

12. The sensor (80) according to any of claims 8 to 11, **characterised in that** the at least one heating element comprises a heating element (520) of an electrically conducting material, located between the first and second structures.

13. The sensor (80) according to any of claims 1 to 11, **characterised in that** the at least one heating element comprises two heating elements (220A, 220B; 420A, 420B; 720A, 720B) of an electrically conducting material, located on either side of a stack including the first and the second structures.

14. The sensor (80) according to claim 13, **characterised in that** the heating elements (220A, 220B; 420A, 420B; 720A, 720B) of the different pixels form together heating tapes, distributed on either side of said stack, and disposed face to face in pairs.

15. The sensor (80) according to any of claims 1 to 14, **characterised in that** each pixel also includes an adhesive portion (93), located between the first and second structures.
